# EUROPEAN PATENT SPECIFICATION

(11) **EP 1 641 440 B1**
(45) Date of publication and mention of the grant of the patent: **23.06.2010**
(21) Application number: 04740278.9
(22) Date of filing: 24.06.2004
(51) Int. Cl.: A61K 9/20, A61K 31/405

(54) **TABLET COMPRISING FLUVASTATIN AND CARMELLOSE CALCIUM**
TABLETTE ENTHALTEND FLUVASTATIN UND CALCIUMCARMELLOSE
COMPRIME CONTENANT DE LA FLUVASTATINE ET DE LA CARMELLOSE CALCIQUE

(30) Priority: 25.06.2003 JP 2003181349
(43) Date of publication of application: 05.04.2006
(73) Proprietor: Novartis AG, 4056 Basel (CH); Novartis Pharma GmbH, 1230 Wien (AT)
(72) Inventor: UEHARA, Tomoyuki, Toyonaka-shi, Osaka (JP); TAGUCHI, Kenji, Sakai-shi, Osaka (JP); KITAOKA, Kenichi, Takatsuki-shi, Osaka (JP)
(74) Representative: Leon, Susanna Iris
(86) International application number: PCT/EP2004/006865
(87) International publication number: WO 2005/000276

(56) References cited:
- EP-A- 0 547 000
- DATABASE CHEMABS [Online] CHEMICAL ABSTRACTS SERVICE, COLUMBUS, OHIO, US; 1998, IRIYAMA, KIMISHIGE ET AL: "Discoloration prevention of pharmaceutical preparations, e.g. of fluvastatin sodium, and the stabilized preparations" XP002298726 retrieved from STN Database accession no. 1998:555709 & JP 10 226644 A2 (NOVARTIS A. G., SWITZ.) 25 August 1998 (1998-08-25)

## Description

The present invention relates to a fluvastatin-containing tablet, particularly a fluvastatin-containing tablet which has excellent disintegrating property and good bioavailability.

Fluvastatin has a generic name, R*,S*-(E)-(±)-7-[3-(fluorophenyl)1-(1-methylethyl)-1H-indol-2-yl]-3,5-dihydroxy-6-heptenoic acid, and is known as a compound showing HMG-CoA reductase-preventing activity, i.e. a cholesterol biosynthesis inhibitor. In fact, its sodium salt formulated in a capsule form is presently available on the market as a drug for treatment of hyperlipoproteinemia and atheroscrelosis.

Fluvastatin has characteristic in being of better taste, high hygroscopicity, low solubility in an acidic medium, low stability to heat, moisture or light, non-stability in an acidic aqueous medium, etc. On formulation of a pharmaceutical preparation comprising fluvastatin as an active ingredient is thus required not only to prevent fluvastatin from its direct contact to atmosphere, for instance, by application of a coating film thereto but also to take any appropriate step for stabilization of fluvastatin. For the latter purpose, it is proposed to formulate fluvastatin with an alkaline medium into an appropriate preparation, said medium being capable of keeping an aqueous solution or dispersion of fluvastatin at a pH of 8 as minimum (cf. Patent Literature 1).

Patent Literature: Japanese patent 2,774,037 discloses fluvastatin-containing capsules and fluvastatin containing tablets comprising crosscarmellose sodium. As stated above, fluvastatin is commercially available in a capsule form. From the industrial viewpoint, it is favorable to formulate fluvastatin in a tablet form rather than a capsule form, because a tablet preparation can be manufactured more easily in a lower cost than a capsule preparation. In addition, there is a general tendency in Japan that patients like a tablet preparation better than a capsule preparation. There is thus present a strong demand to tablets comprising fluvastatin, but such demand has not been satisfied up to the present time. One of the reasons therefore may be present in that a capsule preparation comprising fluvastatin as presently available on the market has such good bioavailability as would hardly be achieved with a tablet preparation.

Namely, a pharmaceutical preparation to be administered orally like a capsule or a tablet is disintegrated, dispersed, dissolved or the like in a digestive organ, absorbed through a digestive mucosa into a vascular system, whereby the active ingredient in the preparation reaches an action site. When the active ingredient is identical, its bioavailability is much affected by the disintegrating property of the preparation. It is generally difficult to make the disintegration rate equal between a capsule and a tablet, and the case of fluvastatin is not exceptional. The difference in disintegration rate thus causes the difference in bioavailability, and a capsule and a tablet cannot be considered equally even when the active ingredient and the administration route are the same. This is a remarkable disadvantage in the clinical aspect.

### Technical problem to be solved:

The object of the present invention is to provide a tablet preparation comprising fluvastatin, which shows good bioavailability at least equal to a capsule preparation comprising fluvastatin as presently available on the market with excellent disintegrating property.

### Solution for technical problem:

The above object of the present invention can be attained by using carmellose calcium (carboxymethylcellulose calcium) as a disintegrating agent on formulation of a tablet preparation comprising fluvastatin.

Carmellose calcium is known as one of numerous disintegrating agents including starch, agar powder, carmellose sodium, crystalline cellulose, hydroxypropylmethylcelulose, hydroxypropyl starch, amylose, sodium alginate, dialkylsulfosuccinate, polyoxyethylene sorbitan monofatty acid ester, calcium carbonate, sodium bicarbonate, etc. and actually used for pharmaceutical preparations of various active ingredients. However, what disintegrating agent is to be specifically used can be decided only through comparative tests with other disintegrating agents for each pharmaceutical preparation and each active ingredient.

For instance, carmellose sodium is one of disintegrating agents as frequently used. In fact, its use was investigated in the course of reaching the present invention. Carmellose sodium is close to carmellose calcium in structure and physical properties, but its use does not provide the resulting tablet with a satisfactory disintegrating property and cannot be used in the present invention. The use of carmellose calcium is thus essential in this invention.

However, any other appropriate disintegrating agent in addition to carmellose calcium is not prevented.

The tablet of the present invention can achieve rapid and substantially perfect intestinal absorption of fluvastatin as the active ingredient contained in such tablet. Incorporation of an alkaline medium such as a carbonate on preparation of the tablet is favorable to ensure the stabilization of fluvastatin over a long period of time.

### Practical embodiments of invention:

In the tablet of the present invention, the active ingredient (i.e. medicament) is fluvastatin, which may be in a free form or a salt form insofar as it is pharmaceutically acceptable. As the salt form, there is usually employed the alkali metal salt, especially the sodium salt. The content of fluvastatin in the tablet is usually from about 0.1 to 60 % by weight, preferably from 0.5 to 40 % by weight.

The tablet of this invention can be prepared by applying a per se conventional tableting procedure to a composition comprising fluvastatin and a per se conventional excipient(s) to make a tablet core, preferably followed by applying a per se conventional procedure to the tablet core.

The tablet of the invention is constituted preferably with a tablet core and a coating layer provided on its surface. Formation of the tablet core may be effected by an indirect tableting method or a direct tableting method. The former comprises admixing granules containing the active ingredient obtained by wet or dry granulation process with a lubricating agent(s), a disintegrating agent(s), etc. and tableting the resultant mixture to make tablets. The latter comprises tableting a mixture of the active ingredient with a diluent(s), a lubricating agent(s), a disintegrating agent(s), etc. to make tablets.

As the excipient(s) to be added to the active ingredient, there are usually employed a filler(s), a disintegrating agent(s), a binding agent(s), a lubricating agent(s), etc. The composition forming the tablet core according to the present invention is characteristic in using carmellose calcium as the disintegrating agent. The amount of carmellose calcium is from about 1 to 10 % by weight, preferably from 3 to 8 % by weight, based on the weight of the composition. It is also characteristic that the tablet core is incorporated with a certain specific alkaline medium as a stabilizer, of which the amount is from about 0.1 to 60 % by weight, preferably from 15 to 50 % by weight, based on the weight of the composition.

The term "alkaline medium" (or "base") as herein used is intended to mean one or more pharmaceutically acceptable substances which can give a pH of at least 8, preferably of about 9 to 10, to an aqueous solution or dispersion of the composition which forms the tablet core in this invention. The pH can be determined by taking a unit dosage of the composition containing 20 mg of fluvastatin and dispersing or dissolving such composition in 10 to 100 ml of water. In order to make the alkaline medium exerted a higher stability, fluvastatin and the alkaline medium in the composition is so mixed to make a good contact association. As a matter of course, the alkaline medium should be inert to fluvastatin as the active ingredient.

The alkaline substance to be used as the alkaline medium may be either soluble in water or hardly soluble or substantially insoluble in water. Examples of the water soluble alkaline substance are inorganic carbonates (e.g. sodium carbonate, potassium carbonate, sodium hydrogen carbonate, potassium hydrogen carbonate), alkali metal or alkaline earth metal dibasic phosphates (e.g. sodium dibasic phosphate, potassium dibasic phosphate, calcium dibasic phosphate), trisodium phosphate, etc. and their mixtures.

Examples of the hardly water-soluble or substantially water-insoluble alkaline substances are magnesium oxide, magnesium hydroxide, magnesium carbonate, magnesium hydrogen carbonate, aluminum hydroxide, calcium carbonate, magnesium aluminum hydroxide, tribasic calcium phosphate, etc. and their mixtures.

Among the alkaline substance as exemplified above, preferred are inorganic carbonates and inorganic hydrogen carbonates, specifically sodium carbonate, sodium hydrogen carbonate, calcium carbonate, etc. and their mixtures. Particularly preferred is the one comprising a water soluble alkaline substance(s) and a hardly water-soluble or substantially water-insoluble substance(s), specifically the combination of a water-soluble carbonate with a substantially water-insoluble carbonate such as the combination of sodium hydrogen carbonate and/or sodium carbonate with calcium carbonate.

The alkaline medium is to be contained in the composition of the tablet core in a sufficient amount to provide the aqueous solution or dispersion of the composition with such a pH of at least 8, preferably at least around 10. In general, the content of the alkaline medium in the composition is from about 0.1 to 60 % by weight, especially from 15 to 50 % by weight. For instance, a water-soluble carbonate(s) such as sodium hydrogen carbonate or sodium carbonate may be contained in an amount of about 0.1 to 35 % by weight, preferably of 1 to 15 % by weight. The weight proportion of the water-soluble carbonate(s) and the substantially water-insoluble carbonate(s) is usually from about 2 : 1 to 1 : 40; for instance, the weight proportion of sodium hydrogen carbonate and calcium carbonate is from about 2 : 1 to 1 : 2.

The composition for formation of the tablet can comprise fluvastatin as an active ingredient, carmellose as a disintegrating agent, an alkaline medium as a stabilizer and optionally a conventional additive(s) for convenience of processing.

Examples of the diluent are carbohydrates (e.g. lactose), gelatinized starch (e.g. starch 1500^{R}; Colorcon Corp.), corn starch, dicalcium phosphate, cellulose, microcrystalline cellulose, sugar, sodium chloride, etc. Among them, preferred are lactose, microcrystalline cellulose, gelatinized starch and their mixtures, particularly microcrystalline cellulose.

In order to ensure the disintegrating and compressing properties of the tablet, the use of carmellose calcium as a disintegrating agent is essential in the present invention. When desired, crystalline cellulose (Avicel^{R} ; FMC Corp.), gelatinized starch, etc. may be additionally incorporated for the same purpose as above.

In general, a binding agent(s) is used for imparting an appropriate binding property to the composition for formation of a tablet. Examples of the binding agent are starches, dextrin, arabic gum, sodium alginate, gelatin, methylcellulose, carboxymethylcellulose, hydroxyethylcellulose, polyvinylpyrrolidone, lactose, mannitol, microcrystalline cellulose, calcium lactate, etc. In the present invention, the use of a binding agent(s) is not essential, because a desirable binding property is obtainable by the use of other components.

As the flowing agent(s), there may be used synthetic silicon dioxide, talc, etc. in an amount of about 0.1 to 10 % by weight, preferably of 0.5 to 6 % by weight. As the lubricating agent(s), there is usable any one optionally chosen from conventional lubricating agents such as starch, magnesium stearate, calcium stearate, sucrose, salt, etc., and the use of magnesium stearate is the most suitable in the present invention. The amount of the lubricating agent is usually from about 0.1 to 3 % by weight, preferably from 0.5 to 1.5 % by weight.

By the use of a composition comprising fluvastatin and additives, there are provided tablet cores of standard unit dosages for oral administration such as 5 mg, 10mg, 20 mg, 30 mg, etc.

The tablet core is preferably applied with any proper coating. For instance, enteric coating may be applied to prevent fluvastatin from its decomposition with gastric juice on the way up to the absorption site of small intestine. Examples of the material usable for enteric coating are hydroxypropylmethylcellulose phthalate, cellulose acetate phthalate, polyvinyl acetate phthalate, methylcellulose phthalate, methacrylic acid/methyl methacrylate copolymer (Eudragit^{R}; Rohm Pharma). The enteric coating may be applied to such an extent as resulting in a weight increase of about 5 to 12 % by weight, preferably of 8 to 10 % by weight on the basis of the weight of the tablet core.

For protecting the tablet of the present invention from discoloration due to moisture and light and also for concealing the bitter taste of the active ingredient, it is desirable to apply coating onto the surface of the tablet. For this purpose, a composition for enteric coating may comprise additionally a clouding agent or a coloring agent. Alternatively, the tablet after application of enteric coating thereto may be further subjected to coating to make an ordinary non-transparent film on the surface.

A film coating composition applicable to the tablet of this invention may comprise a film forming agent chosen from polyethylene glycol, polyvinylpyrrolidone, polyvinyl alcohol, hydroxypropylcellulose, hydroxymethylcellulose, hydroxypropylmethylcellulose, etc. Of these, hydroxypropylmethylcellulose (Opadry^{R}; Colorcon Corp.) is preferred. As the water-philic film forming agent which may be applied using an organic solvent vehicle, there are exemplified ethylcellulose, cellulose acetate, polyvinyl alcohol/maleic anhydride copolymer, etc. The film coating composition may be applied in such an amount as producing a weight increase of about 1 to 10 % by weight, preferably of 2 to 6 % by weight on the basis of the weight of the tablet core.

Examples of other components usable for an enteric or film coating composition include plasticizers such as polyethylene glycol (e.g. polyethylene glycol 6000), triethyl citrate, diethyl phthalate, propylene glycol and glycerol, butyl phthalate, clouding agents such as titanium dioxide, coloring agents such as iron oxide and aluminum flake, etc. These components may be used within ordinary ranges.

Enteric or film coating may be carried out by a per se conventional procedure in an appropriate coating pan or a fluidizing apparatus using water and/or organic solvents such as alcohols (e.g. methanol, ethanol, isopropanol), ketones (e.g. acetone, methylethylketone), chlorinated hydrocarbons (e.g. methylene chloride, dichloroethane), etc.

The composition for formation of the tablet core may comprise usually the following materials (the weight % being based on the weight of the composition): fluvastatin as the active ingredient, about 0.1 to 60 % by weight (preferably 0.5 to 40 % by weight); carmellose as the disintegrating agent, about 1 to 10 % by weight (preferably 3 to 8 % by weight); a carbonate(s) (e.g. sodium hydrogen carbonate + precipitated calcium carbonate) as the stabilizer, about 0.1 to 60 % by weight (preferably 10 to 40 % by weight); microcrystalline cellulose as the diluent, about 1 to 65 % by weight (preferably 50 to 60 % by weight); talc as the flowing agent, about 0.1 to 10 % by weight (preferably 0.5 to 6 % by weight); magnesium stearate as the lubricating agent, about 0.1 to 3 % by weight (preferably 0.5 to 1.5 % by weight).

For preparation of the composition to be used for production of the tablet, fluvastatin and the alkaline medium are first mixed together by dry process or wet process (using a small amount of water or any liquid medium). The resultant mixture is further admixed with other additives to make a uniform mixture. This uniform mixture is subjected to tableting by direct or indirect process. In the course of the above processing, drying may be effected if desired.

A specific embodiment is to mix the active ingredient, the alkaline medium and optionally any other additives with a small amount of water to make granules, followed by drying. The dried granules are admixed with other additives as kept separately or remaining, particularly a diluent to make a mixture suitable for tableting.

In another example of the process using a solvent which assists drying on a fluidizing bed, the active ingredient and the alkaline medium are mixed in a wet state according to a per se conventional procedure and subjected to wet granulation with a certain amount of a diluent. The thus prepared granules are dried, admixed with the remaining diluent and any other component such as a lubricating agent and subjected to tableting.

The drying is carried out conveniently by tray drying or fluidizing bed drying, preferably by the latter. Typically, the drying is effected at an entrance temperature of about 50°C and an RH of not more than 50 %.

On preparation of the composition, it is preferred to subject the active ingredient and the additives except the lubricating agent to sieving with a filter of 30 to 40 mesh prior to wet granulation. Specifically, the active ingredient is first sieved and admixed with a diluent as sieved. The dried granules are sieved through a filter of 18 to 20 mesh and admixed with additives as separately kept.

The composition to be tableted is typically subjected to sieving through a finer filter, e.g. of 24 mesh, combined with a lubricating agent and compressed. The above sieving step generally requires further drying step, whereby the wet granules obtained by granulation are dried to 6 to 8 % L.O.D.. The resultant granules are sieved through a filter of 12 to 14 mesh and dried again to 2 to 3 % L.O.D.

A separate process for said wet granulation technique comprises freeze-drying of the active ingredient and the alkaline medium together from this aqueous solution.

As stated above, the enteric coating and/or film coating composition can be applied to the dosage form as stated above for any particular effect.

The enteric or film coating of the tablet core comprising microcrystalline cellulose with a film coating composition based on water is carried out desirably at a bed temperature of 30 to 50 °C, an entrance temperature of 50 to 80 °C and a relative humidity (RH) of not more than 50 %. In order to attain an optimum stability of the tablet as the final product, it is important that the dosage form after application of the enteric and/or film coating is dried to a water content of not more than 4 %, preferably of not more than 3 %.

The dosage form of the tablet as prepared should be protected from oxidation induced by heat or light and staining with moisture during the storage.

The tablet prepared by the use of the composition of the invention has wonderful storage stability.

The tablet of film coating according to the invention has a disintegrating time of about 10 to 30 minutes. The tablet of enteric coating has generally a disintegrating time of about 60 minutes to 6 hours.

Practical embodiments of the present invention are illustratively shown by the following Examples, which are not to be understood to limit the technical scope of the invention.

### Example

### Example 1

### (1) Preparation of tablet core comprising fluvastatin

a) For preparation of about 600,000 fluvastatin-containing tablets (each tablet containing 30 mg of fluvastatin), fluvastatin sodium, precipitated calcium carbonate, sodium hydrogen carbonate, crystalline cellulose (Avicel PH101) and carmellose calcium (ECG-505) are weighed as shown in Table 1 or Table 1 Bis and mixed together. The resultant mixture is pulverized to a powder mixture.

**Table 1**

| Components | Weight per tablet | Weighed amount |
|---|---|---|
| | (mg) | (kg) |
| Fluvastatin sodium | 31.59 | 18.594 |
| Precipitated calcium carbonate | 42.00 | 25.200 |
| Sodium hydrogen carbonate | 19.800 | 19.800 |
| Crystalline cellulose | 36.540 | 36.540 |
| Carmellose calcium | 5.400 | 5.400 |

**Table 1 Bis**

| Components | Weight per tablet |
|---|---|
| | (mg) |
| Fluvastatin sodium | 31.59 |
| Precipitated calcium carbonate | 42.00 |
| Sodium hydrogen carbonate | 33.00 |
| Crystalline cellulose | 60.90 |
| | |
| Carmellose calcium | 9.00 |

b) The powder mixture as above obtained is mixed by the use of a vertical granulator while agitation for 1 minute, purified water (16 kg) is added thereto in about 3 minutes, and agitation for kneading is effected for additional 1 minute. The kneaded material is crushed with a power mill, and the crushed material is transferred with suction to a fluidizing bed dryer, followed by drying until the water activity value becomes below 0.2 (drying at an air feeding temperature of 60°C for about 50 minutes and an air feeding temperature of 55°C for about 30 minutes). The dried granules are sieved with a vibration sieving machine (30 mesh), and the remaining material on the sieve is crushed with a power mill (30 mesh), followed by sieving again with a vibration sieving machine (30 mesh). The remaining material is further crushed with a power mill (30 mesh), and the crushed material is sieved (30 mesh) to obtain a granulated product.
c) The granulated product as obtained in b), talc and magnesium stearate are weighed as shown in Table 2 (corresponding to 580,000 tablets). Then, talc and magnesium stearate passed through a 42 mesh sieve are added to 5 kg of the granulated product and premixed in a polyethylene-made bag. The resultant mixture and the remainder of the granulated product are charged into a double conical mixer (570 L) and mixed at 21 rpm for 3 minutes to give the granules, which are tableted by the use of a rotary tableting machine to obtain tablet cores containing fluvastatin sodium and having the characteristic properties as shown in Table 3.

**Table 2**

| Components | Weight per tablet | Weighed amount |
|---|---|---|
| | (mg) | |
| Granules for tableting | 176.49 | 102.36 kg |
| Talc | 2.55 | 1479.0 g |
| Magnesium stearate | 0.96 | 556.8 g |

**Table 3**

| Characteristic properties of tablet | |
|---|---|
| Diameter | 7.5 mm |
| Curvature radius | 15 mm |
| Weight per tablet | 180 mg |
| Hardness | 35 N or more |
| Disintegration rate | within 10 minutes |

### (2) Film coating of tablet core

a) Hydroxypropylmethylcellulose 2910, polyethylene glycol (Macrogol 6000), titanium oxide, talc and yellow ferric oxide are weighed as shown in Table 4.

**Table 4**

| Components | Weight per tablet | Weighed amount |
|---|---|---|
| | (mg) | (g) |
| | | |
| Hydroxypropylmethylcellulose | | |
| 2910 | 7.2 | 5760 |
| Macrogol 6000 | 0.8 | 640 |
| Titanium oxide | 1.4 | 1120 |
| Talc | 1.2 | 960 |
| Yellow ferric oxide | 0.2 | 160 |

b) Hydroxypropylmethylcellulose 2910 and Macrogol 6000 are added to purified water (60.8 kg) and stirred to dissolve. The resultant solution is added with talc and stirred to make a suspension (base suspension), which is accommodated in a container. Titanium oxide and yellow ferric oxide are dispersed in purified water (4.8 kg), and the resulting suspension (sunproofing agent suspension) is filtered through a 200 mesh sieve and added to the base suspension to give a mixture. The container and the sieve are washed with purified water (6.4 kg), and the washing solution is added to said mixture, followed by stirring to give a film-forming liquid for undercoating.
c) Hydroxypropylmethylcellulose 2910, polyethylene glycol (Macrogol 6000), titanium oxide, talc and yellow ferric oxide are weighed as shown in Table 5. Hydroxypropylmethylcellulose 2910 and Macrogol 6000 are added to purified water (20.0 kg), followed by stirring for dissolving to obtain a face coating liquid.

**Table 5**

| Components | Weight per tablet | Weighed amount |
|---|---|---|
| | (mg) | (g) |
| | | |
| Hydroxypropylmethylcellulose | | |
| 2910 | 1.0 | 800 |
| Macrogol 6000 | 0.2 | 160 |

d) Into a coating apparatus (Hicoater HC-130), the tablet cores obtained in (1) - c) are charged and pre-heated with intermittent driving to reach an exhaustion temperature of 50°C. The film-forming liquid for undercoating as obtained in above b) is sprayed onto the tablet cores (coating amount: 10.8 mg per tablet) under the conditions set forth below. After the coating is finished, the undercoated tablet is dried in a coating pan for 15 hours. After completion of the drying, the face coating liquid is applied thereon under the same condition as set forth below but with an air feeding temperature on drying of 45°C, a spray speed of 75 mL/min (x 2) and a coating amount of 1.2 mg per tablet. After the coating is finished, drying is effected in a coating pan for 15 hours to obtain the objective tablets containing 30 mg of fluvastatin per tablet.

### Coating conditions

Pressure of spraying air: 5 kg/cm²
Spray speed: 75 mL/min (x 2)
Air feeding temperature: 70°C (for spraying); 45°C (for drying)
Exhaustion temperature: 45°C

### Example 2

In the same manner as in Example 1, tablets containing 10 or 20 mg of fluvastatin are prepared according to the prescription as shown in Table 6.

**Table 6**

| | Components | Tablet (20 mg) | Tablet (10 mg) |
|---|---|---|---|
| | | (mg) | (mg) |
| Tablet | Fluvastatin sodium | 21.06 | 10.53 |
| core | Precipitated calcium carbonate | 22.00 | 11.00 |
| | Sodium hydrogen carbonate | 28.00 | 14.00 |
| | Crystalline cellulose | 40.60 | 20.30 |
| | Carmellose calcium | 6.00 | 3.00 |
| | Talc 1.70 0.85 | | |
| | Magnesium stearate | 0.64 | 0.32 |
| Coating | Hydroxypropylmethylcellulose 2910 | 6.15 | 4.10 |
| film | Macrogol 6000 | 0.75 | 0.50 |
| | Titanium oxide | 1.05 | 0.70 |
| | Talc | 0.90 | 0.60 |
| | Yellow ferric oxide | 0.15 | 0.10 |
| | Total | 129.00 | 66.00 |

### Experiment 1

### Test on biological equivalency between tablets and capsules in human beings

One tablet of the present invention (Example 1; containing fluvastatin 30 mg) or one capsule of "Lochol^{R}" (Commercial product marketed by Novartis Pharma; containing fluvastatin 30 mg) was administered orally once to each of 24 volunteers of healthy human male adults in hunger according to the crossover method with suspension of medication for one week. Comparison of the behavior of fluvastatin in plasma was then made.

The 90 % reliance range of the difference in the average of AUCo-sh (logarithm conversion value) was between log(0.88821) and log(1.18200), which is within the standard range (log(0.8) ∼ log(1.25)). Cmax was greatly varied and, according to the 90 % reliance range method, out of said standard range. The difference in the average of Cmax was log(1.09089), which is within the standard range (log(0.90) ∼ log(1.11)). Said great variation of Cmax is probably due to receipt of the strong initial passage effect by fluvastatin and may be attributed not to the nature of the preparation but to the nature of fluvastatin itself. From the above test results and the results of dissolution test as previously obtained, the tablet and the capsule were decided to be biologically equivalent.

### Experiment 2

### Examination on biological equivalency between tablets and capsules in dissolution

According to the "Guideline for examination of biological equivalency of generic product" attached to Notice No. 487 issued by the Deliberative Council of Medicine on December 22, 1997, examination was carried out under the following conditions at 50 rpm (in case of ii), additionally at 100 ppm): i) pH 1.2 (1st liquid in the disintegration test); ii) pH 5.5 (diluted McIlvaine buffer); iii) pH 6.8 (2nd liquid in the disintegration test) and iv) using water.

### (1) pH 1.2

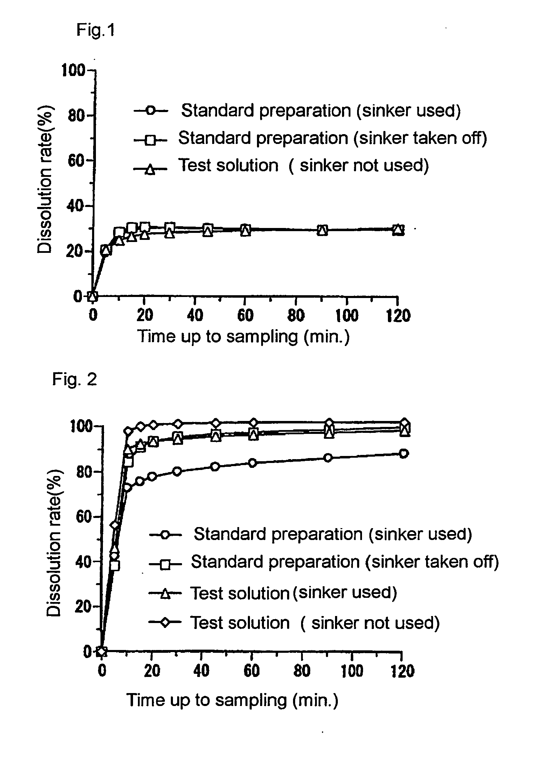

In case of a test solution at pH 1.2, fluvastatin was low in stability and poor in stability. Therefore, the dissolution rates of the test preparation (the tablet of the invention containing 30 mg fluvastatin) and the standard preparation (the "Lochol" capsule containing 30 mg fluvastatin) remained at about 30 %, and no material difference was observed in the average dissolution curve. Also, any influence by the sinker was not observed. According to the judgment standard in the Guideline, the average dissolution rates of the test preparation at 5 minutes and 120 minutes were within those of the standard preparation ± 8 %, and both preparations were judged as equivalent (cf. Fig. 1).

### (2) pH 5.5 (50 rpm)

i) Comparison was made between the standard preparation using a sinker and the test preparation not using a sinker at 5 minutes and 60 minutes corresponding respectively to the average dissolution rates of the standard preparation being 40 % and 85 %. Said preparations were determined to be not equivalent, because the average dissolution rate of the test solution at 60 minutes was out of a range of the average dissolution rate of the standard solution ± 15 %.
ii) Comparison was made between the standard solution removing a sinker in the course of examination and the test preparation not using a sinker. Said preparations were determined to be equivalent, because a dissolution of not less than 85 % within 15 minutes was observed on each of them.
iii) Comparison was made between the standard solution using a sinker and the test solution using a sinker. Said preparations were determined to be equivalent, because the average dissolution rates of the test solution at 5 minutes and 60 minutes corresponding respectively to those of the standard preparation being 40 % and 85 % were each within a range of the average dissolution rate of the standard preparation ± 15 %.

From the above results, the dissolution behaviors of the standard preparation and the test preparation can be considered to be equivalent, because it is appropriate to carry out comparison under the same conditions including the use or non-use of a sinker (cf. Fig. 2).

### (3) pH 6.8, water (50 rpm), pH 5.5 (100 rpm)

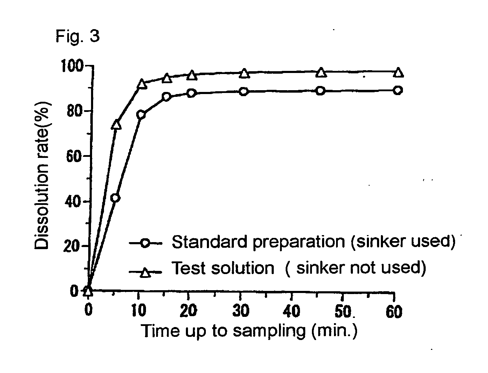

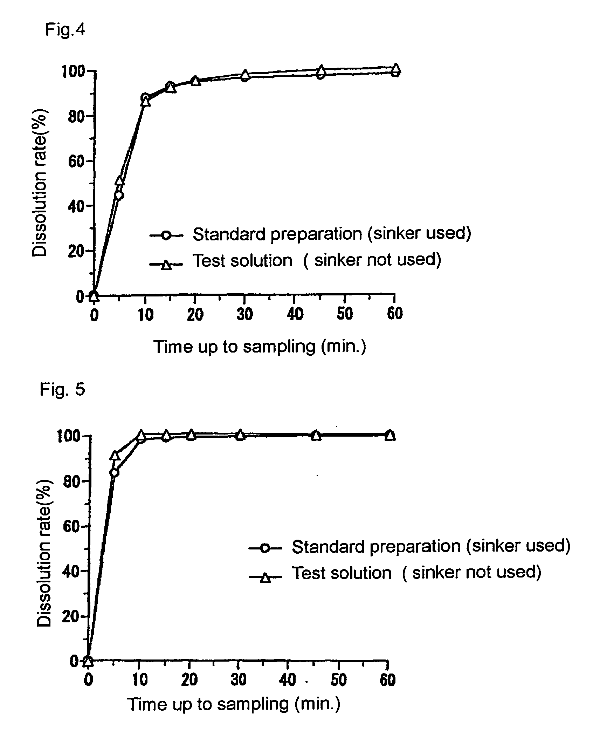

The standard preparation and the test preparation were determined to be equivalent, because a dissolution of not less than 85 % within 15 minutes was observed for both of them (cf. Fig. 3 to 5).

### Experiment 3

### (Method)

In the same manner as in Example 1 (1) and (2), tablet cores (each core weighing 180 mg) were prepared using fluvastatin sodium (30 mg as fluvastatin) and carmellose calcium (ECG505) or crosscarmellose sodium (AcDiSol; FMC BioPolymer Corp.) as shown in Table 7. Each tablet core was subjected to dissolution test according the paddle method (rotation rate, 50 rpm) using water (37°C).

The test cores (A), (B), (C) and (D) were subjected to moisture treatment in a desiccator (room temperature, 100 % RH, 1.5 hours). Then, the dissolution test was effected with those test cores under the same conditions as above.

**Table 7**

| Disintegrating | Tablet core of the invention | | Tablet core for control | |
|---|---|---|---|---|
| Agent | (A) | (B) | (C) | (D) |
| ECG505 | 5.4 mg (3%) | 9.0 mg (5%) | — | — |
| (% by weight)*) | | | | |
| AcDiSol | — | — | 5.4 mg (3%) | 9.0 mg (5 %) |
| (% by weight)*) | | | | |

| | | | | |
|---|---|---|---|---|
| Note: *) Amount per tablet core | | | | |

### (Results)

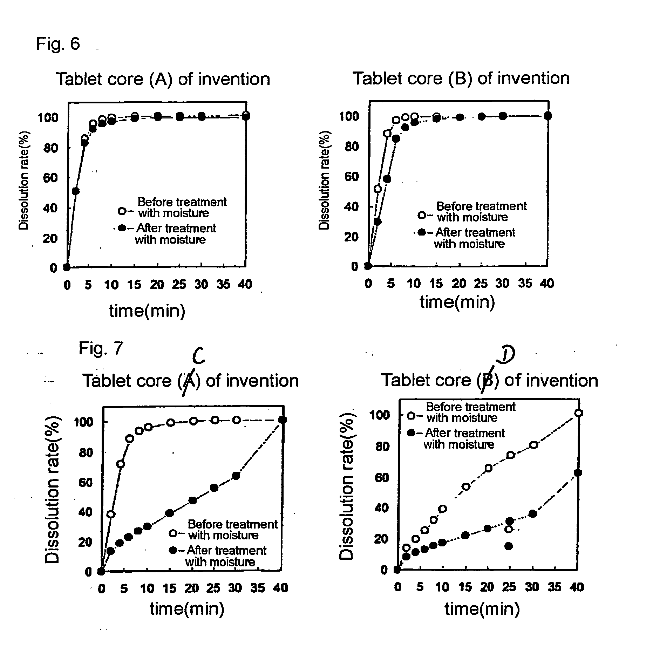

As understood from the dissolution curve in Fig. 6, the tablet cores (A) and (B) (before moisture treatment) of the invention gave good dissolution rates (dissolution rates of not less than 85 % in 15 minutes from the start of examination). As understood from the dissolution curve in Fig. 7, the control tablet core (C) (before moisture treatment) showed a dissolution behavior like the tablet core of the invention but the control tablet core (D) took not less than 30 minutes until the dissolution rate reaches 85 % or more.

Also, the tablet cores (A) and (B) of the invention showed good dissolution rates (dissolution rates of not less than 85 % in 15 minutes from the start of the start of examination) as seen in Fig. 6. To the contrary, the control tablet cores (C) and (D) showed a significant delay of dissolution after moisture treatment in comparison with dissolution before moisture treatment as seen in Fig. 7.

### Technical Effect

As understood from the above, the present invention can provide fluvastatin-containing tablets having a good biological availability equivalent to that of fluvastatin-containing capsules which are marketed commercially. In general, table preparations can be manufactured more efficiently and economically in comparison with capsule preparations. In addition, tablet preparations are more popular to patients than capsule preparations. Accordingly, the present invention makes it possible to administer fluvastatin orally to human beings more economically and conveniently.

### BRIEF EXPLANATION OF DRAWINGS:

Fig. 1 shows the average dissolution curves of "Lochol" (capsule containing 30 mg of fluvastatin) (standard preparation) and of the tablet of the invention (tablet containing 30 mg of fluvastatin)(test preparation) (paddle method; 50 rpm; test solution: pH 1.2, 900 ml; n = 12).
Fig. 2 shows the average dissolution curves of "Lochol" (capsule containing 30 mg of fluvastatin) (standard preparation) and of the tablet of the invention (tablet containing 30 mg of fluvastatin) (test preparation) (paddle method; 50 rpm; test solution: pH 5.5, 900 ml; n = 12).
Fig. 3 shows the average dissolution curves of "Lochol" (capsule containing 30 mg of fluvastatin) (standard preparation) and of the tablet of the invention (tablet containing 30 mg of fluvastatin) (test preparation) (paddle method; 50 rpm; test solution: pH 6.8, 900 ml; n = 12).
Fig. 4 shows the average dissolution curves of "Lochol" (capsule containing 30 mg of fluvastatin) (standard preparation) and of the tablet of the invention (tablet containing 30 mg of fluvastatin) (test preparation) (paddle method; 50 rpm; test solution: water, 900 ml; n = 12).
Fig. 5 shows the average dissolution curves of "Lochol" (capsule containing 30 mg of fluvastatin) (standard preparation) and of the tablet of the invention (tablet containing 30 mg of fluvastatin) (test preparation) (paddle method; 100 rpm; test solution: pH 5.5, 900 ml; n = 12).
Fig. 6 is a graph showing the dissolution test results of the tablet cores of the invention prepared as in Example 1 (1) and (2) (before and after moisture treatment).
Fig. 7 is a graph showing the dissolution test results of the tablet cores for control prepared as in Example 1 (1) and (2) (before and after moisture treatment).

## Claims

1. A fluvastatin-containing tablet which is **characterized in** comprising carmellose calcium as a disintegrating agent.

2. The tablet according to claim 1, which comprises a pharmaceutically acceptable alkaline medium which is capable of affording a pH of at least 8 to an aqueous solution or dispersion of said tablet.

3. The tablet according to claim 2, wherein the alkaline medium is a carbonate.

4. The tablet according to any of claims 1 to 3, which comprises crystalline cellulose as a diluent.

5. The tablet according to any of claims 1 to 4, which has a coating applied thereon.

6. Fluvastatin tablet according to claims 1-5 comprising:
| | |
|---|---|
| Fluvastatin sodium | 31.59 mg |
| Precipitated calcium carbonate | 42.00 mg |
| Sodium hydrogen carbonate | 19.800 mg |
| Crystalline cellulose | 36.540 mg |
| Carmellose calcium | 5.400 mg |

7. Fluvastatin tablet according to claims 1-6 comprising:
| | |
|---|---|
| Fluvastatin sodium | 31.59 mg |
| Precipitated calcium carbonate | 42.00 mg |
| Sodium hydrogen carbonate | 19.800 mg |
| Crystalline cellulose | 36.540 mg |
| Carmellose calcium | 5.400 mg |
| Talc | 2.55 mg |
| Magnesium stearate | 0.96 mg |

8. Fluvastatin tablet according to claims 1-5 comprising:
| | |
|---|---|
| Fluvastatin sodium | 21.06 mg |
| Precipitated calcium carbonate | 22.00 mg |
| Sodium hydrogen carbonate | 28.00 mg |
| Crystalline cellulose | 40.60 mg |
| Carmellose calcium | 6.00 mg |
| Talc | 1.70 mg |
| Magnesium stearate | 0.64 mg |
| Hydroxypropylmethylcellulose 2910 | 6.15 mg |
| Macrogol 6000 | 0.75 mg |
| Titanium oxide | 1.05 mg |
| Talc | 0.90 mg |
| Yellow ferric oxide | 0.15 mg |

9. Fluvastatin tablet according to claims 1-5 comprising:
| | | |
|---|---|---|
| Tablet | Fluvastatin sodium | 10.53 mg |
| core | Precipitated calcium carbonate | 11.00 mg |
| | Sodium hydrogen carbonate | 14.00 mg |
| | Crystalline cellulose | 20.30 mg |
| | Carmellose calcium | 3.00 mg |
| | Talc | 0.85 mg |
| | Magnesium stearate | 0.32 mg |
| Coating | Hydroxypropylmethylcellulose 2910 | 4.10 mg |
| film | Macrogol 6000 | 0.50 mg |
| | Titanium oxide | 0.70 mg |
| | Talc | 0.60 mg |
| | Yellow ferric oxide | 0.10 mg |

10. Fluvastatin tablet according to claims 1-9 **characterized in that** it is has a disintegration time of 10 to 30 minutes.

## Patentansprüche

1. Fluvastatin enthaltende Tablette, die **dadurch gekennzeichnet ist, dass** sie Carmellosecalcium als Zerfallshilfsmittel umfaßt,

2. Tablette nach Anspruch 1, die ein pharmazeutisch akzeptables alkalisches Medium umfasst, das in der Lage ist, bei einer wässrigen Lösung oder Dispersion der Tablette einen pH-Wert von mindestens 8 zu liefern.

3. Tablette nach Anspruch 2, wobei das alkalische Medium ein Carbonat ist,

4. Tablette nach einem der Ansprüche 1 bis 3, die kristalline Cellulose als Verdünnungsmittel umfasst.

5. Tablette nach einem der Ansprüche 1 bis 4, die einen Überzug aufweist, der darauf appliziert ist.

6. Fluvastatintablette nach den Ansprüchen 1 bis 5, die die folgenden Bestandteile umfasst:
| | |
|---|---|
| Fluvastatinnatrium | 31,59 mg |
| Gefälltes Calciumcarbonat | 42,00 mg |
| Natriumhydrogencarbonat | 19,800 mg |
| Kristalline Cellulose, | 36,540 mg |
| Carmellosecalcium | 5,400 mg. |

7. Fluvastatintablette nach den Ansprüchen 1 bis 6, die die folgenden Bestandteile umfasst:
| | |
|---|---|
| Fluvastatinnatrium | 31,59 mg |
| Gefälltes Calciumcarbonat | 42,00 mg |
| Natriumhydrogencarbonat | 19,800 mg |
| Kristalline Cellulose | 36,540 mg |
| Carmellosecalcium | 5,400 mg |
| Talkum | 2,55 mg |
| Magnesiumstearat | 0,96 mg. |

8. Fluvastatintablette nach den Ansprüchen 1 bis 5, die die folgenden Bestandteile umfasst:
| | |
|---|---|
| Fluvastatinnatrium | 21,06 mg |
| Gefälltes Calciumcarbonat | 22,00 mg |
| Natriumhydrogencarbonat | 28,00 mg |
| Kristalline Cellulose | 40,60 mg |
| Carmellosecalcium | 6,00 mg |
| Talkum | 1,70mg |
| Magnesiumstearat | 0,64 mg |
| Hydroxypropylmethylcellulose 2910 | 6,15 mg |
| Macrogol 6000 | 0,75 mg |
| Titanoxid | 1,05 mg |
| Talkum | 0,90 mg |
| Gelbes Eisen(III)oxid | 0,15 mg. |

9. Fluvastatintablette nach den Ansprüche 1 bis 5, die die folgenden Bestandteile umfasst
| | | |
|---|---|---|
| Tablettenkern | Fluvastatinnatrium | 10,53 mg |
| | Gefälltes Calciumcarbonat | 11,00 mg |
| | Natriumhydrogencarbonat | 14,00 mg |
| | Kristalline Cellulose | 20,30 mg |
| | Carmellosecalcium | 3,00 mg |
| | Talkum | 0,85 mg |
| | Magnesiumstearat | 0,32 mg |
| Beschichtungsfilm | Hydroxypropylmethylcellulose 2910 | 4,10 mg |
| | Macrogol 6000 | 0,50 mg |
| | Titanoxid | 0,70 mg |
| | Talkum | 0,60 mg |
| | Gelbes Eisen(III)oxid | 0,10mg. |

10. Fluvastatintablette nach den Ansprüchen 1 bis 9. **dadurch gekennzeichnet, dass** sie eine Zerfallszeit von 10 bis 30 Minuten aufweist,

## Revendications

1. Comprimé contenant de la fluvastatine, qui est **caractérisé en ce qu'**il comprend de la carmellose calcique comme agent de désagrégation.

2. Comprimé selon la revendication 1, qui comprend un milieu alcalin pharmaceutiquement acceptable susceptible de donner un pH d'au moins 8 à une solution ou une dispersion aqueuse dudit comprimé.

3. Comprimé selon la revendication 2, dans lequel le milieu alcalin est un carbonate.

4. Comprimé selon l'une quelconque des revendications 1 à 3, qui comprend de la cellulose cristalline comme diluant.

5. Comprimé selon l'une quelconque des revendications 1 à 4. sur lequel est appliqué un enrobage.

6. Comprimé de fluvastatine selon les revendications 1-5 comprenant:
| | |
|---|---|
| Fluvastatine sodique | 31,59 mg |
| Carbonate de calcium précipité | 42,00 mg |
| Hydrogénocarbonate de sodium | 19,800 mg |
| Cellulose cristallise | 36,540 mg |
| Carmellose calcique | 5,400 mg |

7. Comprimé de fluvastatine selon les revendications 1-6 comprenant:
| | |
|---|---|
| Fluvastatine sodique | 31,59 mg |
| Carbonate de calcium précipité | 42,00 mg |
| Hydrogénocarbonate de sodium | 19,800 mg |
| Cellulose cristalline | 36,540 mg |
| Carmellose calcique | 5,400 mg |
| Talc | 2,55 mg |
| Stéarate de magnésium | 0,96 mg |

8. Comprimé de fluvastatine selon les revendications 1-5 comprenant:
| | |
|---|---|
| Fluvastatine sodique | 21,06 mg |
| Carbonate de calcium précipité | 22,00 mg |
| Hydrogénocarbonate de sodium | 28,00 mg |
| Cellulose cristalline | 40,60 mg |
| Carmellose calcique | 6,00 mg |
| Talc | 1,70 mg |
| Stéarate de magnésium | 0,64 mg |
| Hydroxypropylméthylcellulose 2910 | 6,15 mg |
| Macrogol 6000 | 0,75 mg |
| Oxyde de titane | 1,05 mg |
| Talc | 0,90 mg |
| Oxyde ferrique jaune | 0,15 mg |

9. Comprimé de fluvastatine selon les revendications 1-5 comprenant:
| | | |
|---|---|---|
| Noyau de comprimé | Fluvastatine sodique | 10,53 mg |
| | Carbonate de calcium précipité | 11,00 mg |
| | Hydrogénocarbonate de sodium | 14,00 mg |
| | Cellulose cristalline | 20,30 mg |
| | Carmellose calcique | 3,00 mg |
| | Talc Stéarate de magnésium | 0,85 mg 0,32 mg |
| Film d'enrobage | Hydroxypropylméthylcellulose 2910 | 4,10 mg |
| | Macrogol 6000 | 0,50 mg |
| | Oxyde de titane | 0,70 mg |
| | Talc | 0,60 mg |
| | Oxyde ferrique jaune | 0,10 mg |

10. Comprimé de fluvastatine selon les revendications 1-9, **caractérisé en ce qu'**il possède un temps de désagrégation de 10 à 30 minutes.
